(19) **Europäisches Patentamt / European Patent Office / Office européen des brevets**

(11) **EP 4 481 446 B1**

(12) **EUROPEAN PATENT SPECIFICATION**

(45) Date of publication and mention
of the grant of the patent:
**05.08.2026 Bulletin 2026/32**

(21) Application number: **24212476.6**

(22) Date of filing: **14.06.2021**

(51) International Patent Classification (IPC):
**B65D 6/14** *(2006.01)*    **C07C 265/14** *(2006.01)*
**C08G 18/76** *(2006.01)*    **G02B 1/04** *(2006.01)*

(52) Cooperative Patent Classification (CPC):
(C-Sets available)
**C07C 263/18; C08G 18/246; C08G 18/3876;
C08G 18/7642; C09D 163/00; G02B 1/041** (Cont.)

(54) **XYLYLENE DIISOCYANATE-CONTAINING CONTAINER, METHOD FOR STORING XYLYLENE DIISOCYANATE, AND METHOD FOR TRANSPORTING XYLYLENE DIISOCYANATE**

XYLYLENE DIISOCYANATE-HOLDING CONTAINER, METHOD FOR STORING XYLYLENE DIISOCYANATE, AND METHOD FOR TRANSPORTING XYLYLENE DIISOCYANATE

RÉCIPIENT CONTENANT DU DIISOCYANATE DE XYLYLÈNE, PROCÉDÉ DE STOCKAGE DE DIISOCYANATE DE XYLYLÈNE ET PROCÉDÉ DE TRANSPORT DE DIISOCYANATE DE XYLYLÈNE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **18.06.2020 JP 2020105488**

(43) Date of publication of application:
**25.12.2024 Bulletin 2024/52**

(62) Document number(s) of the earlier application(s) in
accordance with Art. 76 EPC:
**21824852.4 / 4 116 208**

(73) Proprietor: **Mitsui Chemicals, Inc.
Tokyo 104-0028 (JP)**

(72) Inventors:
• **KAWAGUCHI, Masaru
Fukuoka, 836-8610 (JP)**
• **SATO, Yasuaki
Fukuoka, 836-8610 (JP)**
• **SHIRAI, Mai
Fukuoka, 836-8610 (JP)**

(74) Representative: **Mewburn Ellis LLP
Aurora Building
Counterslip
Bristol BS1 6BX (GB)**

(56) References cited:
**EP-A- 3 404 053    JP-A- 2018 177 273**

**(Cont. next page)**

(52) Cooperative Patent Classification (CPC): (Cont.)

C-Sets
**C07C 263/18, C07C 265/14;**
**G02B 1/041, C08L 75/04, C08L 81/00**

**Description**

The present invention relates to a xylylene diisocyanate-containing container, a method for storing a xylylene diisocyanate, and a method for transporting a xylylene diisocyanate.

BACKGROUND ART

It has been considered that an optical lens requiring excellent transparency is formed from a poly(thio)urethane resin. As a material for the optical lens made from the poly(thio)urethane resin, a xylylene diisocyanate composition containing a xylylene diisocyanate has been known (ref: for example, Patent Document 1). Patent Document 2 describes xylylene diisocyanate compositions and optical lenses using the same. Patent Document 3 describes a container containing pentamethylene diioscyanate and a method of preserving the same.

Citation List

Patent Document

Patent Document 1: International Patent Publication No. WO2018/190290
Patent Document 2: EP 3404053A1
Patent Document 3: JP 2018177273A

SUMMARY OF THE INVENTION

PROBLEM TO BE SOLVED BY THE INVENTION

However, when the xylylene diisocyanate composition described in Patent Document 1 is accommodated in a metal container, and stored and transported, the xylylene diisocyanate composition may be colored particularly in a case of a long period of time of storage and transportation. In addition, when the poly(thio)urethane resin is produced using the xylylene diisocyanate composition accommodated in the metal container, the poly(thio)urethane resin may be colored.
The present invention provides a xylylene diisocyanate-containing container having excellent low coloring properties, a method for storing a xylylene diisocyanate, and a method for transporting a xylylene diisocyanate.

MEANS FOR SOLVING THE PROBLEM

In one aspect, the present invention includes a xylylene diisocyanate-containing container comprising:

a xylylene diisocyanate, and
a container accommodating the xylylene diisocyanate,

wherein a resin layer is provided on an inner surface of the container, and the resin layer contains an epoxy phenol resin.
In another aspect, the present invention includes a xylylene diisocyanate-containing container comprising:

a xylylene diisocyanate having an acid content of below 15 ppm, and
a container accommodating the xylylene diisocyanate,

wherein a zinc phosphate coating is provided on an inner surface of the container.
In another aspect, the present invention provides a method for storing a xylylene diisocyanate, the method comprising:
a step of storing the xylylene diisocyanate in a container provided with a resin layer containing an epoxy phenol resin on an inner surface.
In another aspect, the present invention provides a method for transporting a xylylene diisocyanate, the method comprising:
a step of storing and transporting a xylylene diisocyanate in a container provided with a resin layer containing an epoxy phenol resin on an inner surface.
Another embodiment of the present invention includes a method for transporting a xylylene diisocyanate, the method including storing and transporting a xylylene diisocyanate having an acid content of below 15 ppm in a first container provided with a zinc phosphate coating on an inner surface.

The method above may include a step of measuring an acid content of a xylylene diisocyanate, and a step of accommodating the xylylene diisocyanate in a second container provided with a resin layer on an inner surface when the measured acid content is 15 ppm or more and accommodating the xylylene diisocyanate in the first container when the measured acid content is below 15 ppm.

EFFECT OF THE INVENTION

According to the xylylene diisocyanate-containing container of the present invention, since the epoxy phenol resin layer is provided on the inner surface of the container, it is possible to suppress coloring of the xylylene diisocyanate even when the xylylene diisocyanate is accommodated in the container, and stored and transported over a long period of time. Further, it is possible to suppress the coloring of a poly(thio)urethane resin produced from the xylylene diisocyanate.

According to the xylylene diisocyanate-containing container of the present invention, since the zinc phosphate coating is provided on the inner surface of the container, it is possible to suppress the coloring of the xylylene diisocyanate even when the xylylene diisocyanate having an acid content of below 15 ppm is accommodated in the container, and stored and transported over a long period of time. Further, it is possible to suppress the coloring of the poly(thio)urethane resin produced from the xylylene diisocyanate.

According to the method for storing a xylylene diisocyanate of the present invention, since the xylylene diisocyanate is stored in the above-described container, it is possible to suppress the coloring of the poly(thio)urethane resin, while suppressing the coloring of the xylylene diisocyanate.

According to the method for transporting a xylylene diisocyanate of the present invention, since the xylylene diisocyanate is stored and transported in the above-described container, it is possible to suppress the coloring of the poly(thio)urethane resin, while suppressing the coloring of the xylylene diisocyanate.

BRIEF DESCRIPTION OF THE DRAWINGS

FIG. 1 shows a schematic view illustrating one embodiment of a xylylene diisocyanate-containing container of the present invention.

FIG. 2 shows a graph illustrating a correlation between a storage period of a xylylene diisocyanate composition and a color hue (APHA) of the xylylene diisocyanate composition of Example 1 and Comparative Example 1.

FIG. 3 shows a graph illustrating a correlation between a storage period of a xylylene diisocyanate composition and a yellow index (Y.I.) of the xylylene diisocyanate composition of Example 1 and Comparative Example 1.

FIG. 4 shows a graph illustrating a correlation between a storage period of a xylylene diisocyanate composition and b* of the xylylene diisocyanate composition of Example 1 and Comparative Example 1.

FIG. 5 shows a graph illustrating a correlation between a storage period of a xylylene diisocyanate composition and Y.I. of an optical lens produced from the xylylene diisocyanate composition of Example 1 and Comparative Example 1.

FIG. 6 shows a graph illustrating a correlation between a storage period of a xylylene diisocyanate composition and Y.I. of an optical lens produced from the xylylene diisocyanate composition of Examples 3 and 4.

FIG. 7 shows a graph illustrating a correlation between a storage period of a xylylene diisocyanate composition and Y.I. of an optical lens produced from the xylylene diisocyanate composition of Examples 5 and 6.

DESCRIPTION OF EMBODIMENTS

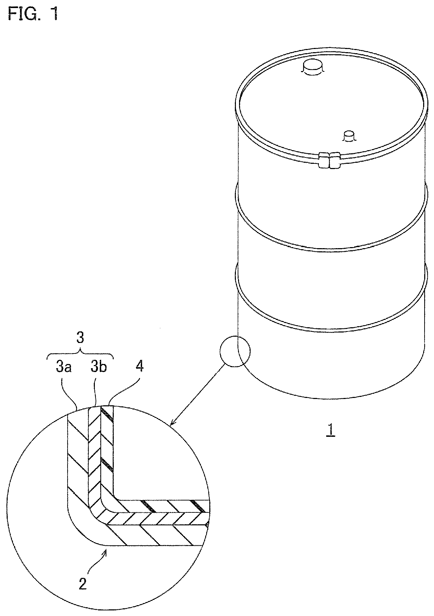

As shown in FIG. 1, a xylylene diisocyanate-containing container 1 includes a xylylene diisocyanate (hereinafter, referred to as an XDI) and a container 2 accommodating the XDI.

Examples of the XDI accommodated in the container 2 include 1,2-XDI (o-XDI), 1,3-XDI (m-XDI), and 1,4-XDI (p-XDI).

These XDIs may be used alone or in combination of two or more.

Of the XDIs, preferably 1, 3-XDI (m-XDI) is used.

The XDI can be also obtained, for example, as a commercially available product (for example, MR-7A etc., manufactured by Mitsui Chemicals, Inc.), and can be also produced by a known method. Examples of the known method include a gas phase method in which a vaporized xylylene diamine (hereinafter, referred to as an XDA) reacts with phosgene, a hydrochloride method in which hydrochloride of the XDA reacts with the phosgene, a one-stage method in which the XDA directly reacts with the phosgene in one stage, and a cold and heat two-stage method in which the XDA reacts with the phosgene at low temperature to be subsequently reacted at high temperature.

Also, the XDI is purified by a known method such as rectification (distillation), extraction, if necessary.

The purity of the XDI is, for example, 95% by mass or more, preferably 98% by mass or more, more preferably 99% by mass or more, further more preferably 99.5% by mass or more, particularly preferably 99.9% by mass or more, and for example, 100% by mass or less. The purity of the XDI can be measured in conformity with the method described in Examples to be described later.

When the purity of the XDI is below 100% by mass, another component is mixed in the XDI. In this case, the container 2 accommodates an XDI composition containing the XDI and the other component.

Examples of the other component include chloromethylbenzyl isocyanate, dichloromethylbenzyl isocyanate, and hydrolyzable chlorine (HC).

A content ratio of the chloromethylbenzyl isocyanate to the total mass of the XDI composition before being accommodated in the container 2 is, for example, 0.2 ppm or more, preferably 6 ppm or more, more preferably 100 ppm or more, and for example, 5000 ppm or less, preferably 4000 ppm or less, more preferably 3000 ppm or less, particularly preferably 1600 ppm or less, especially preferably 1000 ppm or less. The content ratio of the chloromethylbenzyl isocyanate can be measured in conformity with the method described in the [0377] paragraph of Japanese Patent No. 6373536 (hereinafter, the same applies).

The content ratio of the dichloromethylbenzyl isocyanate to the total mass of the XDI composition before being accommodated in the container 2 is, for example, 0.1 ppm or more, preferably 0.3 ppm or more, more preferably 0.6 ppm or more, further more preferably 1.0 ppm or more, and for example, 60 ppm or less, preferably 50 ppm or less, more preferably 30 ppm or less, further more preferably 20 ppm or less, even more preferably 10 ppm or less, even more preferably 5 ppm or less. The content ratio of the dichloromethylbenzyl isocyanate can be measured in conformity with the method described in the [0375] and [0376] paragraphs of Japanese Patent No. 6373536 (hereinafter, the same applies).

The concentration of the hydrolyzable chlorine (HC) with respect to the total mass of the XDI composition before being accommodated in the container 2 is, for example, 10 ppm or more, preferably 20 ppm or more, more preferably 30 ppm or more, and for example, 1000 ppm or less, preferably 500 ppm or less, more preferably 200 ppm or less. The concentration of the hydrolyzable chlorine (HC) is measured in conformity with the method for determining the hydrolyzable chlorine described in JIS K-1603-3 (2007) (hereinafter, the same applies).

An acid content of the XDI composition before being accommodated in the container 2 is, for example, 3000 ppm or less, preferably 2000 ppm or less, more preferably 1000 ppm or less, further more preferably 100 ppm or less, even more preferably 50 ppm or less, even more preferably 30 ppm or less, even more preferably below 15 ppm.

A lower limit value of the acid content of the XDI composition before being accommodated in the container 2 is not limited. The acid content of the XDI composition before being accommodated in the container 2 is, for example, 1 ppm or more.

The acid content is measured by the method described in Examples to be described later (hereinafter, the same applies).

The XDI composition may contain only one kind or two or more kinds of the other components.

Examples of the container 2 include various shaped containers such as funnel cans, drum cans, pail cans, and canister cans, and preferably, funnel cans and drum cans are used. FIG. 1 shows a drum can-shaped container 2.

Further, a size of the container 2 is not particularly limited, and various sizes are used. Specifically, the volume of the container 2 is, for example, 0.5 L or more, preferably 1 L or more, more preferably 16 L or more, and for example, 20000 L or less, preferably 250 L or less.

A material for the container 2 usually includes iron and/or zinc. Also, as shown by an enlarged view of FIG. 1, an epoxy phenol resin layer 4 is provided on an inner surface of the container 2. In other words, the container 2 includes a container body 3 and, if necessary, the resin layer 4. Specifically, in a case of the container 2 accommodating the XDI composition having an acid content of 15 ppm or more, the container 2 includes the resin layer 4. In a case of the container 2 accommodating the XDI composition having an acid content of below 15 ppm, the container 2 may not include the resin layer 4 or may include the resin layer 4. In such cases, the container has a zinc phosphate coating provided on an inner surface of the container.

The container body 3 includes a metal plate 3a which forms the outer shape of the container 2. The container body 3 includes, if necessary, a surface treatment layer 3b laminated on one surface of the metal plate 3a (inner surface of the container 2). Specifically, when the container 2 does not include the resin layer 4, the container body 3 includes the surface treatment layer 3b. The surface treatment layer in such cases includes a zinc phosphate coating. When the container 2 includes the resin layer 4, the container body 3 may not include the surface treatment layer 3b.

The metal plate 3a is not particularly limited, and examples thereof include metal plates containing iron and/or zinc. Specifically, a steel plate is used, more specifically, a carbon steel plate is used, further more specifically, a cold rolling steel plate and a hot rolling steel plate are used.

A thickness of the metal plate 3a is, for example, 0.5 mm or more, preferably 1.0 mm or more, and for example, 2.0 mm or less, preferably 1.6 mm or less.

Examples of the surface treatment layer 3b include chemical conversion coating formed by a known chemical conversion treatment.

The chemical conversion coating is not particularly limited, and examples thereof include iron plating coating, zinc plating coating, tin plating coating, chromium plating coating, aluminum plating coating, nickel plating coating, iron-zinc plating coating, aluminum-zinc plating coating, nickel-zinc plating coating, iron phosphate plating coating, and zinc phosphate plating coating. These chemical conversion coatings may be used alone or in combination of two or more.

Examples of the surface treatment layer 3b usually include coating containing iron and/or zinc. Specifically, iron plating coating, zinc plating coating, iron phosphate coating, and zinc phosphate plating coating are used, more specifically, iron phosphate coating and zinc phosphate plating coating are used, further more specifically, zinc phosphate coatings are used.

In the case where the container 2 does not include the resin layer 4, the container body 3 includes the surface treatment layer 3b comprising a zinc phosphate coating.

The thickness of the surface treatment layer 3b is, for example, 2 $\mu$m or more, preferably 5 $\mu$m or more, and for example, 100 $\mu$m or less, preferably 50 $\mu$m or less.

If necessary, for example, a coating layer or a primer layer may be also formed on one surface of the metal plate 3a (inner surface of the container 2), the other surface of the metal plate 3a (outer surface of the container 2), one surface of the surface treatment layer 3b (inner surface of the container 2), an interface between the metal plate 3a and the surface treatment layer 3b.

The resin layer 4 is provided on the inner surface of the container body 3. The resin layer 4 is laminated on the inner surface of the container body 3. In FIG. 1, the resin layer 4 is provided on the surface at the opposite side to the metal plate 3a in the surface treatment layer 3b. The resin layer 4 is formed on the inner surface of the container body 3 by laminating the resin.

The resin includes an epoxy phenol resin. This may be in combination with polyolefin resins (for example, polyethylene resin, polypropylene resin, cyclic polyolefin resin, etc.), AS (acrylonitrile styrene) resins, ABS (acrylonitrile butadiene styrene) resins, polyvinyl chloride resins, fluorine-based resins, polyester resins (for example, polyethylene terephthalate resin, polyethylene naphthalate resin, etc.), phenol resins, polyacrylic resins, other epoxy resins (for example, epoxy amine resins, etc.), polyimide resins, polyamide resins (for example, various nylon, polyamideimide resin, etc.), poly(thio)urethane resins, cellulose-based resins, silicone-based resins, and polycarbonate resins.

The poly(thio)urethane resin includes a polyurethane resin and a polythiourethane resin. The polyurethane resin is a reaction product of isocyanate and polyol. The polythiourethane resin is a reaction product of isocyanate and polythiol.

These resins may be used alone or in combination of two or more.

As the resin, an epoxy phenol resin is used, preferably, an epoxy phenol resin and an epoxy amine resin are used. In other words, the resin layer 4 contains an epoxy phenol resin, more preferably contains an epoxy phenol resin and an epoxy amine resin, particularly preferably consists of an epoxy phenol resin.

When the container includes the epoxy phenol resin layer 4, it is possible to stably suppress coloring of the XDI composition accommodated in the container 2 and it is possible to stably suppress the coloring of the poly(thio)urethane resin produced from the XDI composition accommodated in the container 2.

A method for forming the resin layer 4 is not particularly limited, and a known method is used.

The resin layer 4 is formed by coating the above-described resin on the inner surface of the container body 3, for example, by a known resin coating method such as spray coating method, dip coating method, electrostatic coating method, and powder coating method.

Further, the resin layer 4 is also formed by laminating a film (single-layer film, multiple layer film) of the above-described resin on the inner surface of the container body 3 by a known resin laminate method such as dry laminate and hot melt laminate.

The thickness of the resin layer 4 is, for example, 5 $\mu$m or more, preferably 10 $\mu$m or more, and for example, 1000 $\mu$m or less, preferably 500 $\mu$m or less.

Specific examples of the container 2 include epoxy phenol coating cans, and preferably, an epoxy phenol coating can and an epoxy amine coating can are used.

Although not shown, it is also possible to improve gas barrier properties and water barrier properties by depositing inorganic oxide etc. in the container 2.

Then, by accommodating and storing the XDI composition (XDI) in the container 2, it is possible to obtain the XDI-containing container 1 having excellent low coloring properties.

Further, when an XDI composition (XDI) having an acid content of 15 ppm or more is stored in a container without the resin layer 4, iron and/or zinc contained in the container body 3 may elute into the XDI composition, and the XDI composition may contain iron and/or zinc. Therefore, it is not preferable that the container 2 without the resin layer 4 accommodates the XDI composition having an acid content of 15 ppm or more.

On the other hand, in the container 2 provided with the epoxy phenol resin layer 4 on the inner surface, even when the XDI composition (XDI) having an acid content of 15 ppm or more is accommodated and stored in the container 2, it is possible to suppress elution of iron and/or zinc into the XDI composition. Therefore, the container 2 provided with the epoxy phenol resin layer 4 on the inner surface can accommodate the XDI composition (XDI) having an acid content of 15 ppm or more. The container 2 provided with the epoxy phenol resin layer 4 on the inner surface can also accommodate the XDI composition (XDI) having an acid content of below 15 ppm.

When the XDI composition having an acid content of below 15 ppm is stored in the container 2 provided with the surface treatment layer 3b without the resin layer 4 on the inner surface, the elution of iron and/or zinc into the XDI composition is suppressed by the surface treatment layer 3b. Therefore, though it is not preferable that the container 2 provided with the surface treatment layer 3b without the resin layer 4 on the inner surface accommodates the XDI composition having an acid content of 15 ppm or more, the container 2 can accommodate the XDI composition having an acid content of below 15 ppm.

The iron content in the XDI composition at the time of storage of being accommodated in the container 2 is, for example, 1 ppb or more, and for example, 1000 ppb or less, preferably 500 ppb or less.

The zinc content in the XDI composition at the time of storage of being accommodated in the container 2 is, for example, 1 ppb or more, and for example, 1000 ppb or less, preferably 500 ppb or less.

Further, the content ratio of the chloromethylbenzyl isocyanate to the total mass of the XDI composition at the time of storage of being accommodated in the container 2 is, for example, 0.2 ppm or more, preferably 6 ppm or more, more preferably 100 ppm or more, and for example, 5000 ppm or less, preferably 4000 ppm or less, more preferably 3000 ppm or less, further more preferably 1600 ppm or less, even more preferably 1000 ppm or less.

The content ratio of the dichloromethylbenzyl isocyanate to the total mass of the XDI composition at the time of storage of being accommodated in the container 2 is, for example, 0.1 ppm or more, preferably 0.3 ppm or more, more preferably 0.6 ppm or more, further more preferably 1.0 ppm or more, and for example, 60 ppm or less, preferably 50 ppm or less, more preferably 30 ppm or less, further more preferably 20 ppm or less, even more preferably 10 ppm or less, particularly preferably 5 ppm or less.

The concentration of the hydrolyzable chlorine (HC) with respect to the total mass of the XDI composition at the time of storage of being accommodated in the container 2 is, for example, 10 ppm or more, preferably 20 ppm or more, more preferably 30 ppm or more, and for example, 1000 ppm or less, preferably 500 ppm or less, more preferably 200 ppm or less.

Further, the XDI-containing container 1 may be sealed with an inert gas such as nitrogen gas, if necessary.

In the XDI-containing container 1, the XDI composition (XDI) is stored in a state of being accommodated in the above-described container 2. In other words, the XDI composition (XDI) having an acid content of 15 ppm or more is stored in the container 2 provided with the resin layer 4 on the inner surface. In addition, the XDI composition (XDI) having an acid content of below 15 ppm is stored in the container 2 provided with a zinc phosphate coating without the resin layer 4 on the inner surface. The XDI composition (XDI) having an acid content of below 15 ppm may be stored in the container 2 provided with the resin layer 4 on the inner surface.

Specifically, when the acid content of the xylylene diisocyanate is measured, and the measured acid content is 15 ppm or more, and when the xylylene diisocyanate is accommodated in the container 2 provided with the resin layer on the inner surface, and the measured acid content is below 15 ppm, the xylylene diisocyanate is accommodated in the container 2 provided with the zinc phosphate coating without the resin layer 4 on the inner surface.

As the storage conditions, a storage temperature is, for example, -5°C or more, preferably 0°C or more, and for example, 50°C or less, preferably 40°C or less, more preferably 30°C or less, further more preferably 25°C or less. In addition, the storage temperature is, for example, 5°C or less, preferably 1°C or less at the time of storage at low temperature.

When the storage temperature is the above-described upper limit or less, it is possible to stably suppress the coloring of the XDI composition.

Also, a storage period is, for example, 1 day or more, preferably 10 days or more, more preferably 1 month or more, further more preferably 3 months or more, particularly preferably 6 months or more, and for example, 3 years or less, preferably 1 year or less.

Also, if necessary, the XDI composition (XDI) is transported in a state of being accommodated in the above-described container 2. In other words, the XDI composition (XDI) having an acid content of 15 ppm or more is stored and transported in the container 2 provided with the resin layer 4 on the inner surface. In addition, the XDI composition (XDI) having an acid content of below 15 ppm is stored and transported in the container 2 provided with the zinc phosphate coating without the resin layer 4 on the inner surface. Further, the XDI composition (XDI) having an acid content of below 15 ppm may be stored and transported in the container 2 provided with the resin layer 4 on the inner surface. The above-described storage temperature can be applied as the temperature during the transportation.

The XDI in the XDI-containing container 1 can be widely used as a material for the poly(thio)urethane resin, and is preferably used, for example, in the production of optical lenses and coatings.

The optical lens is, for example, produced by reaction of the XDI with polythiol. In the production of the optical lenses, for example, a casting method can be used.

Examples of the optical lens include transparent lenses, sunglass lenses, polarizing lenses, spectacle lenses, camera lenses, pick-up lenses, and contact lenses.

The coating is produced, for example, from a two-component curable coating material containing an A agent as a curing agent and a B agent as a main agent. The XDI is available for both the A agent and the B agent.

When the XDI is used for the A agent, the A agent contains, for example, an XDI modified product (for example, isocyanurate modified product, urethane modified product, etc.) which is modified from the XDI and/or an isocyanate group-terminated prepolymer which is a reaction product of the XDI and the polyol.

When the XDI is used for the B agent, the B agent contains, for example, a polyurethane polyol which is a reaction product of the XDI and the polyol.

Examples of the coating include paints and adhesives.

In the above-described XDI-containing container 1, as shown in FIG. 1, the resin layer 4 is provided on the inner surface of the container 2. Therefore, even when the XDI is accommodated in the container 2, and stored and transported over a long period of time, it is possible to suppress the coloring of the XDI (XDI composition). Further, it is possible to suppress the coloring of the poly(thio)urethane resin produced from the XDI (XDI composition).

The resin layer 4 containing the epoxy phenol resin allows further more reliable suppression of the coloring of the XDI (XDI composition).

Examples

Next, the present invention is further described based on Examples, Comparative Example, and Reference Examples below. The present invention is however not limited by these Examples, Comparative Example, and Reference Examples. The specific numerical values in mixing ratio (content ratio), property value, and parameter used in the following description can be replaced with upper limit values (numerical values defined as "or less" or "below") or lower limit values (numerical values defined as "or more" or "above") of corresponding numerical values in mixing ratio (content ratio), property value, and parameter described in the above-described "DESCRIPTION OF EMBODIMENTS". All designations of "part" or "parts" and "%" mean part or parts by mass and % by mass, respectively, unless otherwise particularly specified.

In addition, a measurement method of various properties described below is described as follows.

<Purity of m-XDI in m-XDI Composition>

A calibration curve was prepared from an area value of the obtained gas chromatogram by analyzing an m-XDI having the purity of 99 mol% as a reference material by an internal reference method using gas chromatography under the following conditions.

Next, the m-XDI composition obtained by the rectification was analyzed by the gas chromatography under the following conditions, thereby obtaining the number of moles of the m-XDI. The obtained number of moles was converted into the mass, thereby calculating a content ratio (purity) of the m-XDI in an m-XDI composition. The retention time of an internal reference material was 8.8 minutes, and the retention time of the m-XDI was 13.8 minutes.

Equipment: SHIMADZU GC-2014 (manufactured by Shimadzu Corporation)
Column: DB-1 (film thickness of 1.5 $\mu$m, internal diameter of 0.53 mm$\times$ length of 60 m) (manufactured by Shimadzu Corporation)
Oven temperature: temperature rising from 130°C to 220°C at 3°C/min, and temperature rising to 300°C after reaching 220°C at 10°C/min
Split ratio: pulsed splitless method
Injection port temperature: 280°C
Detector temperature: 300°C
Carrier gas: $N_2$, 158 kPa (constant pressure control)
Internal reference material: 100 mg of 1,2,4,5-tetrachlorobenzene
Solvent: chloroform
Sample concentration: 2.0% by mass of chloroform solution
Injection volume: 2 $\mu$L
Detection method: FID

<NBDI Purity of NBDI Composition Containing Mixture of 2,5-bis(isocyanatomethyl)bicyclo[2.2.1]heptane and 2,6-bis(isocyanatomethyl)bicyclo[2.2.1]heptane (hereinafter, referred to as NBDI)>

A calibration curve was prepared from an area value of the obtained gas chromatogram by analyzing an NBDI having the purity of 99 mol% as a reference material by an internal reference method using gas chromatography under the following conditions.

Next, an NBDI composition obtained by the rectification was analyzed by the gas chromatography under the following conditions, thereby obtaining the number of moles of the NBDI. The obtained number of moles was converted into the mass, thereby calculating a content ratio (purity) of the NBDI in the NBDI composition. The retention time of an internal reference material was 8.8 minutes, and the retention time of the NBDI was 13.0.

Equipment: SHIMADZU GC-2014 (manufactured by Shimadzu Corporation)
Column: DB-1 (film thickness of 1.5 $\mu$m, internal diameter of 0.53 mm× length of 60 m) (manufactured by Shimadzu Corporation)
Oven temperature: temperature rising from 130°C to 220°C at 5°C/min, and temperature rising to 300°C after reaching 220°C at 20°C/min
Split ratio: pulsed splitless method
Injection port temperature: 280°C
Detector temperature: 300°C
Carrier gas: N$_2$, 95.4 kPa (constant pressure control)
Internal reference material: 100 mg of 1,2,4,5-tetrachlorobenzene
Solvent: chloroform
Sample concentration: 2.0% by mass of chloroform solution
Injection volume: 2 $\mu$L
Detection method: FID

<XDI Composition, Color Hue (APHA) of NBDI Composition>

Measurement was carried out by the method in conformity with JIS K 0071 (1998). Specifically, the APHA was obtained by comparison with a dilution solution of a reference solution having the same level of concentration as the color of a sample using a reference solution prepared by dissolving a reagent of platinum and cobalt. A "degree" thereof was referred to as a measurement value. The smaller the degree, the more excellent the color hue.

<Yellow Index (Y.I.) And b* of XDI Composition>

Measurement was carried out using a color-difference meter CT-210 manufactured by Minolta Co., Ltd. First, distilled water was added to a cell CT-A20 having an optical path length of 20 mm, and white calibration was carried out as Y=100.00, x=0.3101, and y=0.3162. Thereafter, a sample was put into the same cell, thereby measuring chromaticity coordinates x, y, and b*. The Y.I. was calculated by the following formula (1) based on the values of x and y as measurement results.

$$Y.I.=(234×x+106×y+106)/y \qquad (1)$$

The Y.I. was referred to as a numerical value of the color hue of the XDI composition, and the higher the numerical value, the greater the degree of coloring.
When a liquid XDI composition was measured, the measurement was carried out by being put in a cell having a thickness of 10 mm.

<Calculation of Value (Y.I. value) of Yellow Index of Optical Lens (Resin)>

A resin was prepared as a circular flat plate plastic lens having a thickness of 9 mm and a diameter of 75 mm, and the chromaticity coordinates x, y were measured using a color-difference meter CT-210 manufactured by Minolta Co., Ltd.
The Y.I. was calculated by the above-described formula (1) based on the values of x and y as the measurement results.
There was a correlation that the smaller the Y.I. value, the more excellent the color hue of the plastic lens, and the greater the Y.I., the poorer the color hue.

Preparation Example 1: Preparation of First m-XDI Composition (acid content: 15 ppm or more)

An autoclave with a pressure regulator (inner volume of 2 m$^3$) equipped with a reflux cooling tube, a stirring blade, a thermometer, a hydrogen chloride gas introduction tube, a phosgene introduction tube, a material tank, and a material charging pump was used as a reactor. In the reactor, 846 kg of orthodichlorobenzene as an inert solvent was charged, and 136.2 kg (1.0 k mol) of m-xylylenediamine and 621 kg of orthodichlorobenzene were charged in the material tank (total amine concentration of 8.5% by mass).

Next, the temperature in the reactor was increased to 120°C, and thereafter, the internal pressure was adjusted to be higher than the atmospheric pressure by 0.01 MPa. Then, a hydrogen chloride gas was started to be charged from the hydrogen chloride gas introduction tube into the reactor at a rate of 43.8 kg/hr, and at the same time, the m-xylylenediamine diluted with an inert solvent was started to be charged from the material tank at a rate of 379 kg/hr with the material charging pump, and the entire amount thereof was charged over two hours. Thereafter, the mixture was further aged for one hour, while the hydrogen chloride gas was charged at 20 kg/hr.

Next, after the temperature of the reaction solution (hydrochloride slurry) was increased to 160°C in the reactor, phosgene was introduced from a phosgene introduction tube at 100 kg/hr (1.0 k mol/hr) and reacted for eight hours, while the temperature thereof was kept. After completion of the reaction, the unreacted phosgene and the hydrogen chloride gas were removed by purging nitrogen into the reactor. Then, the reaction solution was filtered to remove 0.8 kg (dry weight) of the unreacted hydrochloride. The resulting filtrate was desolvated, thereby obtaining 188.6 kg of m-XDI composition having the m-XDI purity of 98.10%.

Next, the obtained m-XDI composition was rectified, thereby obtaining an m-XDI composition having the m-XDI purity of 99.99% by mass. The obtained m-XDI composition was blown with a hydrochloric acid gas, thereby preparing the m-XDI composition having the acid content of 20 ppm.

The acid content was measured by the following method.

<Measurement Method of Acid Content>

A sample (20 g) was accurately weighed into a 200-ml beaker containing a stirrer, and 100 ml of a solvent (mixture of acetone and ethanol at a ratio of 1 to 1 (volume ratio)) was added thereto to be placed on a hot plate. The resulting sample was dissolved by heating, and thereafter, was reacted, while stirred at room temperature for 10 to 20 minutes.

Next, by using an automatic titrator (manufactured by HIRANUMA Co., Ltd., COM-500), an N/100 methanolic potassium hydroxide solution (in conformity with JIS K4101, prepared using methanol, prepared by diluting 0.1 mol/L of the standardized methanolic potassium hydroxide accurately by 10 times) was titrated, and an inflection point of the obtained titration curve was used as an end point. A blank test was carried out for the test under the same conditions.

The acid content (%) was calculated in accordance with the following formula from the titration results.

$$\text{Formula: acid content} = [0.0365 \times (A-B) \times f]/S$$

In the above-described formula, A was a used amount (ml) of the N/100 methanolic potassium hydroxide solution required for titrating the sample, B was a used amount (ml) of the N/100 methanolic potassium hydroxide solution required for the blank test, f was a factor of the N/100 methanolic potassium hydroxide solution, and S was the weight (g) of the sample.

Preparation Example 2: Preparation of Second m-XDI Composition (acid content: below 15 ppm)

An m-XDI composition having the m-XDI purity of 99.99% by mass was obtained in the same manner as in Preparation Example 1. A hydrochloric acid gas was blown into the obtained m-XDI composition, thereby preparing the m-XDI composition having the acid content of 2 ppm.

Preparation Example 3: Preparation of NBDI Composition

The same reactor as that in Preparation Example 1 was used. In the reactor, 958 g of orthodichlorobenzene was charged as a reaction solvent, and 154.2 g (1.0 mol) of a mixture of 2,5-bis(aminomethyl)bicyclo[2.2.1]heptane and 2,6-bis(aminomethyl)bicyclo[2.2.1]heptane, and 702 g of orthodichlorobenzene were charged into a material tank (total amine concentration of 8.5% by mass).

Next, the temperature in the reactor was increased to 120°C, and thereafter, the inside of the autoclave was adjusted to be higher than the atmospheric pressure by 0.01 MPa. Then, a hydrogen chloride gas was started to be charged from the hydrogen chloride gas introduction tube into the reactor at a rate of 43.8 g/hr, and at the same time, the mixture of the 2,5-bis(aminomethyl)bicyclo[2.2.1]heptane and the 2,6-bis(aminomethyl)bicyclo[2.2.1]heptane diluted with a solvent was started to be charged from the material tank at a rate of 428.1 g/hr with the material charging pump, and the entire amount thereof was charged over two hours. Furthermore, the mixture was aged for one hour, while the hydrogen chloride gas was charged at 20 g/hr.

Next, after the temperature of the hydrochloride slurry was increased to 160°C in the reactor, phosgene was blown from a phosgene introduction tube at 100 g/hr (1.0 mol/hr) and reacted for eight hours, while the temperature thereof was kept. After completion of the reaction, the unreacted phosgene and the hydrogen chloride gas were removed by purging nitrogen into the system. Then, the reaction solution was filtered to remove 0.5 g (dry mass) of the unreacted hydrochloride. The resulting filtrate was desolvated, thereby obtaining 206.9 g of NBDI composition having the NBDI purity of 98.5% by mass.

Next, the obtained NBDI composition was rectified, thereby obtaining the NBDI composition having the NBDI purity of 99.99% by mass.

Examples 1 and 2 and Comparative Example 1

A first m-XDI composition or a second m-XDI composition was charged into the container shown in Table 1 below, and thoroughly sealed with a nitrogen gas to be completely closed.

Thereafter, the resulting product was left to stand under the environment of 20°C for 12 months from the production (0 months).

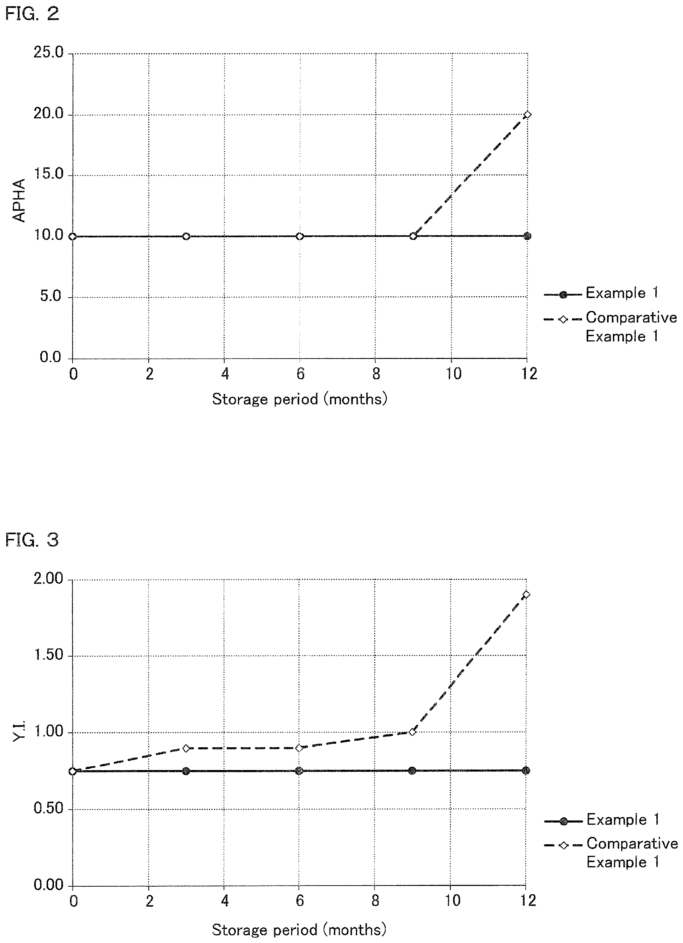

In addition, each container was opened to collect the sample at the timing shown in Table 1. Then, APHA, YI., and b* of each sample (m-XDI composition) were measured. The results are shown in Table 1 and FIGS. 2 to 4.

Further, an optical lens (polythiourethane resin) was prepared by reacting the sample (m-XDI composition) collected at the timing shown in Table 1 with 4-mercaptomethyl-1,8-dimercapto-3,6-dithiaoctane (active hydrogen compound). Specifically, in a sufficiently dried flask, 36.4 g of m-XDI composition, 0.001 g of dibutyltin dichloride, 0.07 g of ZELEC UN (trade name, acid phosphate ester, internal mold release agent, manufactured by Stepan Company), and 0.05 g of BIOSORB 583 (trade name, ultraviolet absorber, manufactured by Sakai Chemical Industry Co., Ltd.) were weighed, and stirred at 25°C for 1 hour to be mixed and dissolved. Thereafter, 33.6 g of 4-mercaptomethyl-1,8-dimercapto-3,6-dithiaoctane was charged and mixed, thereby preparing a liquid mixture (polymerizable composition).

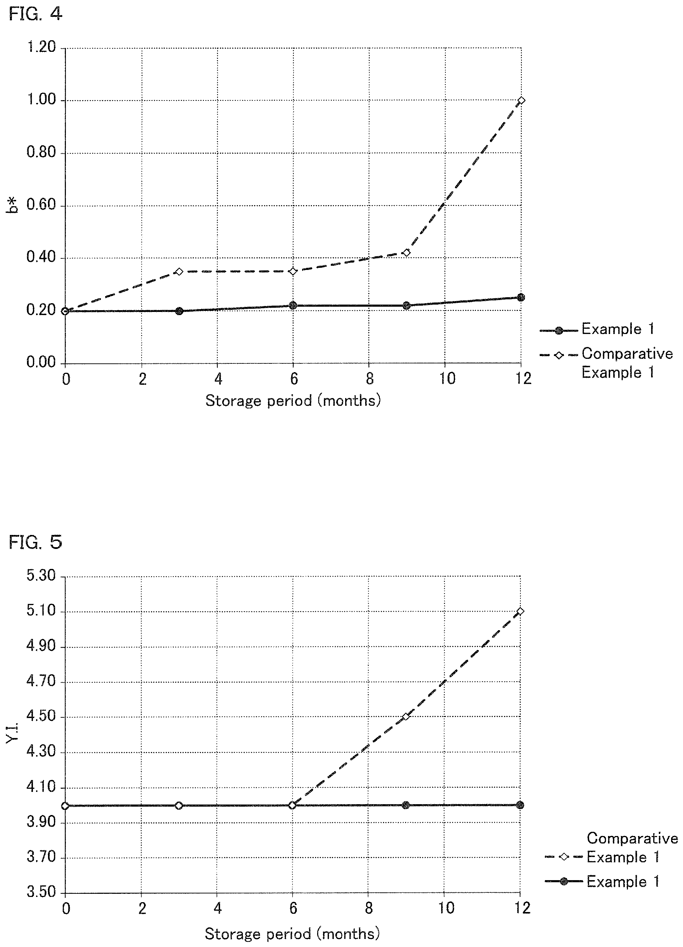

The liquid mixture was defoamed at 600 Pa for 1 hour, and thereafter, filtered through a 3-$\mu$m PTFE filter. Thereafter, the filtered product was injected into a mold consisting of a glass mold and a tape. The mold was put into an oven, and the temperature was gradually increased from 10°C to 120°C to be polymerized for 18 hours. After completion of the polymerization, the mold was taken out from the oven to be released, thereby obtaining a resin. The obtained resin was further annealed at 130°C for 4 hours. The Y.I of the optical lens of the obtained resin was measured. The results are shown in Table 1 and FIG. 5.

It was confirmed that Example 1 in which the container was an epoxy phenol coating can could decrease the APHA, the Y.I., and the b* of the m-XDI composition and decrease the Y.I. of the optical lens as compared with Comparative Example 1 (embodiment in which the acid content was 15 ppm or more and the container was a zinc phosphate treatment drum can). In addition, it was confirmed that Example 2 in which the acid content was below 15 ppm and the container was a zinc phosphate treatment drum can could decrease the APHA of the m-XDI composition as compared with Comparative Example 1.

<Storage Container>

·Container Including Resin Layer

Example 1: epoxy phenol coating can (drum can including the epoxy phenol resin layer on the inner surface of container)

·Container without Resin Layer

Example 2 and Comparative Example 1: zinc phosphate treatment drum can (drum can made of the zinc phosphate treatment steel plate (manufactured by JFE CONTAINER CO., LTD.)

Reference Example 1

An NBDI composition was left to stand to collect the sample in the same manner as in Example 1, except that the m-XDI composition was changed to the NBDI composition. The APHA of each sample (NBDI composition) was measured in the same manner as the description above. The results are shown in Table 1. In Reference Example 1, it was confirmed that when the container was the epoxy phenol can, the APHA of the NBDI composition did not deteriorate.

Reference Example 2

An NBDI composition was left to stand to collect the sample in the same manner as in Comparative Example 1, except that the m-XDI composition was changed to the NBDI composition. The APHA of each sample (NBDI composition) was measured in the same manner as the description above. The results are shown in Table 1. It was confirmed that when the container was the zinc phosphate treatment drum can, the APHA of the NBDI composition did not deteriorate.

[Table 1]

Table 1

| No. | | | | Ex. 1 | Ex. 2 | Comparative Ex. 1 | Reference Ex. 1 | Reference Ex. 2 |
|---|---|---|---|---|---|---|---|---|
| Polyisocyanate Composition | | | | m-XDI (Acid Content of 20 ppm) | m-XDI (Acid Content of 2 ppm) | m-XDI (Acid Content of 20 ppm) | NBDI | NBDI |
| Container | | | | Epoxy Phenol Coating Can | Zinc Phosphate Treatment Drum Can | Zinc Phosphate Treatment Drum Can | Epoxy Phenol Coating Can | Zinc Phosphate Treatment Drum Can |
| Polyisocyanate Composition | APHA | 0 | [Months] | 10.0 | 10.0 | 10.0 | 10.0 | 10.0 |
| | | 3 | | 10.0 | 10.0 | 10.0 | 10.0 | 10.0 |
| | | 6 | | 10.0 | 10.0 | 10.0 | 10.0 | 10.0 |
| | | 9 | | 10.0 | 10.0 | 10.0 | 10.0 | 10.0 |
| | | 12 | | 10.0 | 10.0 | 20.0 | 10.0 | 10.0 |
| | Y.I. | 0 | [Months] | 0.75 | | 0.75 | | |
| | | 3 | | 0.75 | | 0.90 | | |
| | | 6 | | 0.75 | | 0.90 | | |
| | | 9 | | 0.75 | | 1.00 | | |
| | | 12 | | 0.75 | | 1.90 | | |
| | b* | 0 | [Months] | 0.20 | | 0.20 | | |
| | | 3 | | 0.20 | | 0.35 | | |
| | | 6 | | 0.22 | | 0.35 | | |
| | | 9 | | 0.22 | | 0.42 | | |
| | | 12 | | 0.25 | | 1.00 | | |
| Optical Lens | Y.I. | 0 | [Months] | 4.00 | | 4.00 | | |
| | | 3 | | 4.00 | | 4.00 | | |
| | | 6 | | 4.00 | | 4.00 | | |
| | | 9 | | 4.00 | | 4.50 | | |
| | | 12 | | 4.00 | | 5.00 | | |

Examples 3 and 4

An m-XDI composition of a production lot different from the m-XDI compositions used in Examples 1 and 2 and Comparative Example 1 was charged into each of the following containers and thoroughly sealed with a nitrogen gas to be completely closed.
Thereafter, the resulting product was left to stand under the environment of 20°C for 6 months from the production (0 months).
In addition, each container was opened to collect the sample at the timing shown in Table 2. Then, the APHA of each sample (m-XDI composition) was measured. The results are shown in Table 2.

Further, an optical lens (polythiourethane resin) was prepared by reacting the sample (m-XDI composition) collected at the timing shown in Table 2 with pentaerythritol tetrakismercaptopropionate (active hydrogen compound). Specifically, in a sufficiently dried flask, 43.5 g of m-XDI composition, 0.008 g of dibutyltin dichloride, 0.10 g of ZELEC UN (trade name, acid phosphate ester, internal mold release agent, manufactured by Stepan Company), and 0.05 g of BIOSORB 583 (trade name, ultraviolet absorber, manufactured by Sakai Chemical Industry Co., Ltd.) were weighed, and stirred at 25°C for 1 hour to be mixed and dissolved. Thereafter, 56.5 g of pentaerythritol tetrakismercaptopropionate was charged and mixed, thereby preparing a liquid mixture (polymerizable composition).

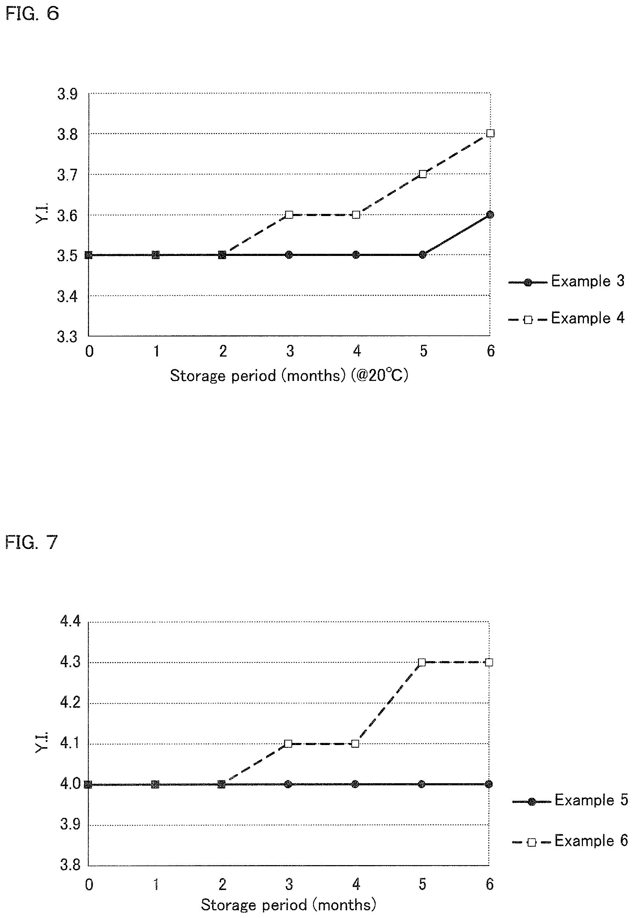

The liquid mixture was defoamed at 600 Pa for 1 hour, and thereafter, filtered through a 3-$\mu$m PTFE filter. Thereafter, the filtered product was injected into a mold consisting of a glass mold and a tape. The mold was put into an oven, and the temperature was gradually increased from 20°C to 120°C to be polymerized for 22 hours. After completion of the polymerization, the mold was taken out from the oven to be released, thereby obtaining a resin. The obtained resin was further annealed at 120°C for 2 hours. The Y.I of the obtained resin was measured. The results are shown in Table 2 and FIG. 6.

It was confirmed that Example 3 in which the container was an epoxy phenol coating can could decrease the Y.I. of the optical lens (resin) as compared with Example 4 (embodiment in which the container was an epoxy amine coating can).

Examples 5 and 6

An m-XDI composition of the same production lot as the m-XDI compositions used in Examples 3 and 4 was charged into each of the following containers and thoroughly sealed with the nitrogen gas to be completely closed.

Thereafter, the resulting product was left to stand under the environment of 20°C for 6 months from the production (0 months).

In addition, each container was opened to collect the sample at the timing shown in Table 2. Then, the APHA of each sample (m-XDI composition) was measured. The results are shown in Table 2.

Further, an optical lens (polythiourethane resin) was prepared by reacting the sample (m-XDI composition) collected at the timing shown in Table 2 with 4-mercaptomethyl-1,8-dimercapto-3,6-dithiaoctane (active hydrogen compound).

Specifically, in a sufficiently dried flask, 36.4 g of m-XDI composition, 0.001 g of dibutyltin dichloride, 0.07 g of ZELEC UN (trade name, acid phosphate ester, internal mold release agent, manufactured by Stepan Company), and 0.05 g of BIOSORB 583 (trade name, ultraviolet absorber, manufactured by Sakai Chemical Industry Co., Ltd.) were weighed, and stirred at 25°C for 1 hour to be mixed and dissolved. Thereafter, 33.6 g of 4-mercaptomethyl-1,8-dimercapto-3,6-dithiaoctane was charged and mixed, thereby preparing a liquid mixture (polymerizable composition).

The liquid mixture was defoamed at 600 Pa for 1 hour, and thereafter, filtered through a 3-$\mu$m PTFE filter. Thereafter, the filtered product was injected into a mold consisting of a glass mold and a tape. The mold was put into an oven, and the temperature was gradually increased from 10°C to 120°C to be polymerized for 18 hours. After completion of the polymerization, the mold was taken out from the oven to be released, thereby obtaining a resin. The obtained resin was further annealed at 130°C for 4 hours. The Y.I of the obtained resin was measured. The results are shown in Table 2 and FIG. 7.

It was confirmed that Example 5 in which the container was an epoxy phenol coating can could decrease the Y.I. of the optical lens (resin) as compared with Example 6 (embodiment in which the container was an epoxy amine coating can).

<Storage Container>

·Container Including Resin Layer

Examples 3 and 5: epoxy phenol coating can (drum can including the epoxy phenol resin layer on the inner surface of the container)

Reference examples 4 and 6: epoxy amine coating can (drum can including the epoxy amine resin layer on the inner surface of the container)

[Table 2]

Table 2

| No. | | | | Ex. 3 | Ex. 4 | Ex. 5 | Ex. 6 |
|---|---|---|---|---|---|---|---|
| Polyisocyanate Composition | | | | m-XDI (Acid Content of 20 ppm) | m-XDI (Acid Content of 20 ppm) | m-XDI (Acid Content of 20 ppm) | m-XDI (Acid Content of 20 ppm) |
| Container | | | | Epoxy Phenol Coating Can | Epoxy Amine Coating Can | Epoxy Phenol Coating Can | Epoxy Amine Coating Can |
| Polyisocyanate Composition | APHA | 0 | [Months] | 10.0 | 10.0 | 10.0 | 10.0 |
| | | 2 | | 10.0 | 10.0 | 10.0 | 10.0 |
| | | 5 | | 10.0 | 10.0 | 10.0 | 10.0 |
| | | 6 | | 10.0 | 10.0 | 10.0 | 10.0 |
| Optical Lens | Y.I. | 0 | [Months] | 3.5 | 3.5 | 4.0 | 4.0 |
| | | 1 | | 3.5 | 3.5 | 4.0 | 4.0 |
| | | 2 | | 3.5 | 3.5 | 4.0 | 4.0 |
| | | 3 | | 3.5 | 3.6 | 4.0 | 4.1 |
| | | 4 | | 3.5 | 3.6 | 4.0 | 4.1 |
| | | 5 | | 3.5 | 3.7 | 4.0 | 4.3 |
| | | 6 | | 3.6 | 3.8 | 4.0 | 4.3 |

Description of Reference Numerals

1    Xylylene diisocyanate-containing container

2    Container

3    Container body

4    Resin layer

**Claims**

1. A xylylene diisocyanate-containing container comprising:

   a xylylene diisocyanate, and
   a container accommodating the xylylene diisocyanate,
   wherein a resin layer is provided on an inner surface of the container, and
   the resin layer contains an epoxy phenol resin.

2. A xylylene diisocyanate-containing container comprising:

   a xylylene diisocyanate having an acid content of
   below 15 ppm measured as indicated in the description, and
   a container accommodating the xylylene diisocyanate,
   wherein a zinc phosphate coating is provided on an inner surface of the container.

3. A method for storing a xylylene diisocyanate, the method comprising: a step of storing the xylylene diisocyanate in a container provided with a resin layer containing an epoxy phenol resin on an inner surface.

4. A method for transporting a xylylene diisocyanate, the method comprising:

a step of storing and transporting a xylylene diisocyanate in a container provided with a resin layer containing an epoxy phenol resin on an inner surface.

**Patentansprüche**

1. Xylylendiisocyanat-enthaltender Behälter, der Folgendes umfasst:

   ein Xylylendiisocyanat und
   einen Behälter, in dem das Xylylendiisocyanat untergebracht ist,
   wobei auf einer inneren Oberfläche des Behälters eine Harzschicht vorgesehen ist und
   die Harzschicht ein Epoxyphenolharz enthält.

2. Xylylendiisocyanat-enthaltender Behälter, der Folgendes umfasst:

   ein Xylylendiisocyanat, das einen Säuregehalt von weniger als 15 ppm aufweist, der wie in der Beschreibung angegeben gemessen wird, und
   einen Behälter, in dem das Xylylendiisocyanat untergebracht ist,
   wobei auf einer inneren Oberfläche des Behälters eine Zinkphosphat-Beschichtung vorgesehen ist.

3. Verfahren zum Lagern eines Xylylendiisocyanats, wobei das Verfahren Folgendes umfasst: einen Schritt des Lagerns des Xylylendiisocyanats in einem Behälter, der auf einer inneren Oberfläche mit einer Harzschicht versehen ist, die ein Epoxyphenolharz enthält.

4. Verfahren zum Transportieren eines Xylylendiisocyanats, wobei das Verfahren Folgendes umfasst: einen Schritt des Lagerns und Transportierens eines Xylylendiisocyanats in einem Behälter, der auf einer inneren Oberfläche mit einer Harzschicht versehen ist, die ein Epoxyphenolharz enthält.

**Revendications**

1. Récipient contenant du diisocyanate de xylylène, comprenant :

   un diisocyanate de xylylène ; et
   un récipient recevant le diisocyanate de xylylène, dans lequel une couche de résine est prévue sur une surface interne du récipient, et
   la couche de résine contient une résine époxy phénol.

2. Récipient contenant du diisocyanate de xylylène, comprenant :

   un diisocyanate de xylylène présentant une teneur en acide inférieure à 15 ppm mesurée comme indiqué dans la description, et
   un récipient recevant le diisocyanate de xylylène, dans lequel un revêtement de phosphate de zinc est prévu sur une surface intérieure du récipient.

3. Procédé de stockage d'un diisocyanate de xylylène, le procédé comprenant :
   une étape de stockage du diisocyanate de xylylène dans un récipient muni d'une couche de résine contenant une résine époxy phénol sur une surface intérieure.

4. Procédé de transport d'un diisocyanate de xylylène, le procédé comprenant :
   une étape de stockage et de transport d'un diisocyanate de xylylène dans un récipient muni d'une couche de résine contenant une résine époxy phénol sur une surface intérieure.

FIG. 1

FIG. 2

FIG. 3

FIG. 4

FIG. 5

FIG. 6

FIG. 7

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2018190290 A
- EP 3404053 A1
- JP 2018177273 A
- JP 6373536 B